(19) Europäisches Patentamt
European Patent Office
Office européen des brevets

(11) **EP 1 408 323 B1**

(12) **EUROPEAN PATENT SPECIFICATION**

(45) Date of publication and mention
of the grant of the patent:
**09.04.2008 Bulletin 2008/15**

(51) Int Cl.:
***G01N 15/14*** *(2006.01)*    ***G06T 7/60*** *(2006.01)*

(21) Application number: **02257052.7**

(22) Date of filing: **10.10.2002**

(54) **Microarray analysis**

Bildanalyse von Mikroarrays

Analyse de microéchatillons

(84) Designated Contracting States:
**AT BE BG CH CY CZ DE DK EE ES FI FR GB GR IE IT LI LU MC NL PT SE SK TR**

(43) Date of publication of application:
**14.04.2004 Bulletin 2004/16**

(73) Proprietor: **BlueGnome Ltd**
**Great Shelford**
**CB2 5LD (GB)**

(72) Inventor: **Haan, Nicholas**
**Adam and Eve St.**
**Cambridge,**
**CB1 1DX (GB)**

(74) Representative: **Haley, Stephen**
**Gill Jennings & Every LLP**
**Broadgate House**
**7 Eldon Street**
**London EC2M 7LH (GB)**

(56) References cited:
**US-A- 6 054 300**

- **HAAN N M ET AL: "Bayesian models for DNA sequencing" 2002 IEEE INTERNATIONAL CONFERENCE ON ACOUSTICS, SPEECH, AND SIGNAL PROCESSING. PROCEEDINGS (CAT. NO.02CH37334), PROCEEDINGS OF INTERNATIONAL CONFERENCE ON ACOUSTICS, SPEECH AND SIGNAL PROCESSING (CASSP'02), ORLANDO, FL, USA, 13-17 MAY 2002, pages IV4020-IV4023 vol.4, XP002238128 2002, Piscataway, NJ, USA, IEEE, USA ISBN: 0-7803-7402-9**
- **HAAN N M ET AL: "Sequential methods for DNA sequencing" 2001 IEEE INTERNATIONAL CONFERENCE ON ACOUSTICS, SPEECH, AND SIGNAL PROCESSING. PROCEEDINGS (CAT. NO.01CH37221), 2001 IEEE INTERNATIONAL CONFERENCE ON ACOUSTICS, SPEECH, AND SIGNAL PROCESSING. PROCEEDINGS, SALT LAKE CITY, UT, USA, 7-11 MAY 2001, pages 1045-1048 vol.2, XP002238129 2001, Piscataway, NJ, USA, IEEE, USA ISBN: 0-7803-7041-4**
- **HAAN N M ET AL: "Modelling electropherogram data for DNA sequencing using variable dimension MCMC" 2000 IEEE INTERNATIONAL CONFERENCE ON ACOUSTICS, SPEECH, AND SIGNAL PROCESSING;ISTANBUL, TURKEY JUN 5-JUN 9 2000, vol. 6, 2000, pages 3542-3545, XP010505662 ICASSP IEEE Int Conf Acoust Speech Signal Process Proc;ICASSP, IEEE International Conference on Acoustics, Speech and Signal Processing - Proceedings 2000 IEEE, Piscataway, NJ, USA**

**Description**

The present invention relates to the analysis of microarray images. In particular it relates to the inclusion of information about the data generation process in models of DNA microarrays in order to improve such analysis, although it can apply to other microarray-based processes.

DNA microarray technology provides a way of measuring the expression of thousands of genes in a sample. DNA microarrays have provided the first industrial means of measuring how gene expression varies between different cells and conditions. They also enable the detection of mutation in the genome at a previously unthinkable speed.

To gain maximum benefit from DNA microarray technology, the analysis of the results obviously needs to be as accurate as possible. However, current methods of analysing DNA microarray images are not refined enough to evaluate gene expression with high accuracy. This means that in order to gain useful results DNA microarray experiments may have to repeated, or other additional experiments performed.

The applicants have appreciated that this lack of accuracy is due to the use of traditional image processing techniques to analyse results and extract information from the results. Traditional image processing techniques are not well suited to this application, especially as they effectively discard valuable information available about the data generation process in the analysis. Traditional image processing techniques do not rely on detailed models of the microarray process but work for example, by detecting sharp transitions. They do not use the fact that probe and target distributions interact in a complicated way to form these spots.

Probe distribution is the distribution of DNA of known sequence in the sample bound to an array. Target distribution is the distribution of DNA in the one or more samples applied to the array. Understanding the probe and target distributions rather than considering the problem as simple spot detection results in significant insights into what should be expected of the data.

Current methods of DNA microarray analysis also do not allow meaningful confidence measures to be assigned to results, thus limiting the usefulness of the results. Current confidence measures are poor and of little use because they do not incorporate a full understanding of the data generation process. They do not satisfactorily tackle the problem of uncertainty specific to fluorescence, target and probe variation.

The present invention aims to improve the accuracy of DNA microarray analysis so that gene expression can be more accurately evaluated. This is particularly useful for low expression levels or subtle expression changes. The present invention also allows absolute expression levels and not just ratios to be measured for all types of microarrays.

The present invention also aims to enable meaningful confidence measures to be assigned to results so that, for example, drug discovery, diagnostics and research decisions can be carried out with confidence.

Additionally, the present invention enables improved reproducibility and automation of microarray experiments.

According to the present invention there is provided a method of analysing microarray images, the method comprising the steps of:

receiving data from a microarray process,
modelling the microarray process to define a microarray model comprising at least one of a target distribution of test and control sample sequences and defining a first independent sub-model and a probe distribution of a known sample sequence and defining a second independent sub-model,
comparing the received data with the microarray model in order to extract information from the data, and
outputting the information.

The data may be received from a detector corresponding to a control target sample and a detector corresponding to a test target sample.

The microarray process may be a DNA microarray process.

The extracted information may be gene expression information.

When at least the second independent sub-model is employed in the modelling step, the second independent sub-model may comprise a model of the spotting process which may include an understanding of how adjacent spots interact.

The modelling step may further comprise modelling the interaction between the background distribution of the received signal and at least one of target distribution and probe distribution. The background distribution may include non-specific hybridisation.

The modelling step may further comprise modelling fluorescence to define a third independent sub-model. The third independent sub-model may include information on the effect of DNA sequence on fluorescence.

The modelling step may further comprise modelling hybridisation to define a fourth independent sub-model. The fourth independent sub-model may include information on the effect of DNA sequence on hybridisation.

The modelling step may further comprise modelling spatial variation of target concentration.

The modelling step may further comprise modelling detector nonlinearity.

The comparing step may further comprise comparing the received image data with the microarray model in order to predict missing data. The missing data may be due to saturation in the device which creates the image data.

The structure of the DNA microarray model may be hierarchical.

According to the present invention there is also provided an apparatus for analysing microarray images, the apparatus comprising:

> means for receiving data from a microarray process,
> means for modelling the microarray process to define a microarray model comprising at least one of a target distribution of test and control sample sequences and defining a first independent sub-model and a probe distribution of a known sample sequence and defining a second independent sub-model,
> means for comparing the received data with the microarray model in order to extract information from the data, and
> means for outputting the information.

The means for modelling may further comprise means for modelling the interaction between the background distribution of the received signal and at least one of target distribution and probe distribution.

The means for modelling may further comprise means for modelling fluorescence to define a third independent sub-model.

The means for modelling may further comprise means for modelling hybridisation to define a fourth independent sub-model.

The means for modelling may further comprise means for modelling spatial variation of target concentration.

The means for comparing may further comprise means for comparing the received image data with the microarray model in order to predict missing data.

The means for modelling may further comprise means for modelling detector nonlinearity.

The present invention includes key information about the data generation process for DNA microarrays in models of the microarray process, therefore allowing better analysis of the results. Previously either the relevance and usefulness of this information has not been appreciated or it has not been thought possible to include the information in models due to its complex mathematical expression or the computing power needed.

An example of the present invention will now be described with reference to the accompanying drawing, in which:

> Figure 1 is a diagram of a comparative hybridisation process with a two channel cDNA array; and
> Figure 2 is a schematic block diagram showing the system of the present invention.

The following discussion refers to cDNA microarrays, but the term microarray in relation to the present invention can also refer to other types of microarrays, such as protein microarrays and macroarrays, Affymetrix GeneChips (RTM) and similar. The invention can also include approaches that do not use a control sample.

In a cDNA microarray experiment, a control sample 1 and a test sample 2 with DNA of known sequence are compared. Typically messenger RNA (mRNA) 4 is extracted 10 from cells 3. The control 1 and test 2 samples are labelled 11 with different fluorescent dyes 5, 6 (usually Cy3 and Cy5), which emit at different wavelengths. Upon application 12 to an array 8, the two samples 1, 2 competitively hybridise to the array 8. Unhybridised DNA 7 is washed away, the fluorescent dyes are excited and a scanner 13 generates image data 9 corresponding to each fluorescent dye.

The image data must then be analysed to extract useful information about gene expression such as a measurement of gene expression or nucleotide polymorphisms. An improved analysis of this image data is enabled by means of the present invention, which compares the image data with improved models of the DNA microarray process.

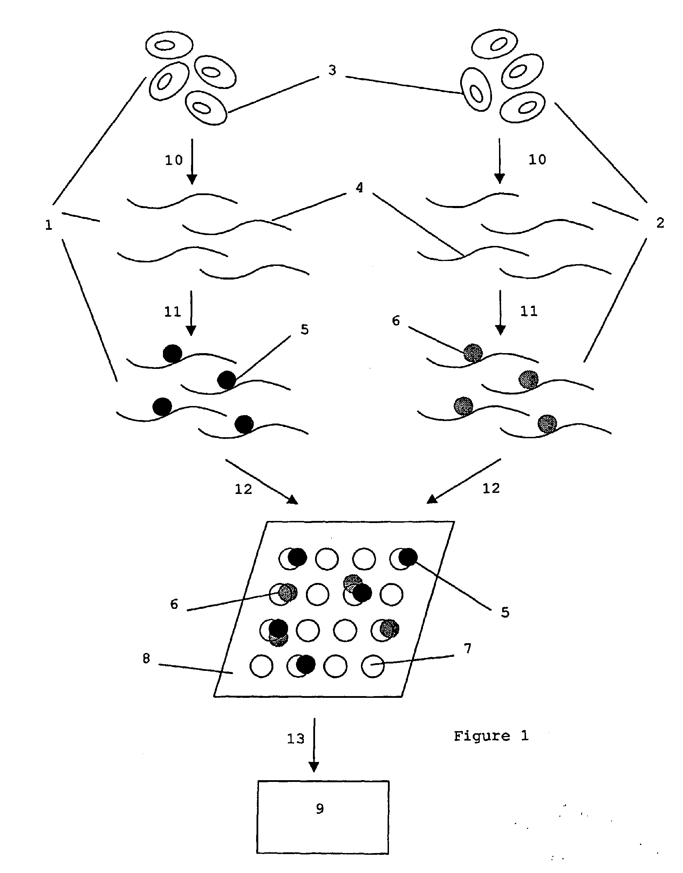

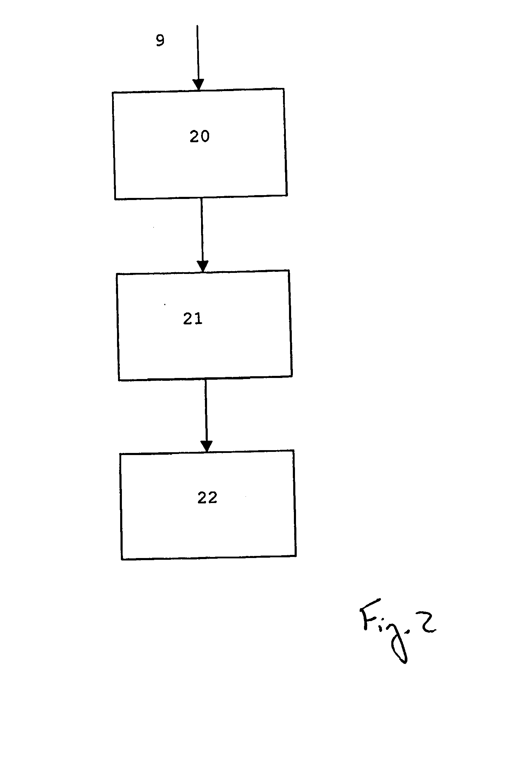

Figure 2 is a schematic diagram of the system of the present invention. The system of the present invention comprises a receiver 20 which receives data, which in this example is image data from a microarray analysis of the type shown in figure 1. A combined modelling and comparator device 21, which may be an appropriately configured PC or processor, generates modelling data and compares the data received by the receiver 20 with the modelling data, in accordance with certain criteria that will be explained in detail below. The comparison is performed to extract information, again as described in more detail below, that can provide confidence measures or other relevant information to an output device 22 which may simply be a display, or which alternatively can be a data recorder.

An alternative use of the present invention is to evaluate the quality of previously analysed data. In this case, the receiver 20 receives analysed image data that has been the result of an analysis by a known mechanism, and which is related to a microarray procedure, and compares such data with the original data and appropriate models created by the modelling and comparator device 21 in order to provide data at the output 22 which is indicative of the quality of the previous analysis.

The modelling processes employed in the system shown in figure 2 will now be described in more detail.

The preparation, spotting and hybridisation processes are modelled on a grid defined by the scanner resolution. These processes typically comprise sample preparation, spotting onto the array, bonding of DNA to the surface of the array, rehydration, denaturation, hybridisation of sample to spotted DNA, and washing of unhybridised sample from the slide. The grid corresponds roughly to the array of pixels that comprise the end image. Within a pixel region, all relevant quantities are assumed constant.

The grid has dimension $M_1$ x $M_2$ where $M_1$ is the width of the image. An individual pixel is denoted

$$m \overset{\Delta}{=} (m_1, m_2) \in (\{1, \ldots, M_1\}, \{1, \ldots, M_2\})$$

The mathematical specifics are now developed in the context of a single spot to maintain notational simplicity. Extension to the multiple spot case is straightforward.

The DNA of known sequence in the sample bound to the slide is referred to as probe sequence. The DNA in the test and control samples is referred to as target sequence. The total probe at a given pixel location before hybridisation is denoted by $d_m$. Available cy3 and cy5 target at each location before hybridisation is denoted by

$$\mathbf{a}_m \overset{\Delta}{=} \{a_{m,cy3}, a_{m,cy5}\}$$

Target DNA can bind to the slide through: specific hybridization to complementary probe, non-specific hybridisation to the surface of the slide (typically to imperfectly blocked regions), and non-specific hybridisation to partially complementary probe sequence. Not all probe is necessarily firmly attached to the slide, and may be dislodged during washing.

With the invention it is assumed the samples are "perfect" and contain no contaminants. It is also assumed that pins are perfectly cleaned before depositing each new sample.

The distribution of probe available for hybridization is influenced most strongly by the platform specific spotting process, whether it be accomplished by inkjet, mechanical pins, or photolithography. A number of other processes can contribute to the distribution, however, including rehydration and denaturation.

The presence of probe is denoted at location m with the indicator variable $I_m \in \{0,1\}$.

The distribution of quantities of total amount of probe at a given pixel location before hybridisation, p ($d_m$), can be given by:

$$p(d_m) = p(I_m = 1|\cdot)p(d_m|I_m = 1,\cdot) + p(I_m = 0|\cdot)\delta(d_m)$$

where the quantity · represents dependence on a range of quantities, some of which are unique to the experimental apparatus in question.

The model for the distribution of indicators incorporates both information about the spotting device such as circularity, and other subsequent effects. The model should be sufficiently flexible to accommodate a range of spotting effects.

The distribution of indicators, p(I), can be given by:

$$p(I_m = 1|_{-m}) = \frac{f(\|m - r_i\|)g(I_{-m})}{f(\|m - r_i\|)g(I_{-m}) + (1 - f(\|m - r_i\|))(1 - g(I_{-m}))}$$

where f(·) is dependent on the shape of the spotting device, and g(·) caters for run-off, separated clumps, and other less ideal effects. Model selection is sensitive to the balance between f(·) and g(·). Both are typically restricted to the range of values between O and 1.

An alternative formulation is:

$$p(I_m = 1|I_{-m}) = w_1 f(\|m - r_i\|) + w_2 g(I_{-m})$$

where f(·) and g(·) retain similar meanings. The weights can be adjusted depending on the perceived importance of spot continuity.

Both f(·) and g(·) can usefully take many forms.

In the invention, in order to reduce computation an assumption of first order symmetric Markovian dependence on adjacent pixels can be useful: $g(I_{-m})=g(I_{\{m\}})=g(\Sigma I_{\{m\}})$

where $I_{\{m\}}$ denotes the neighbourhood of adjacent pixels. In this approach a large number of surrounding on pixels implies a high probability.

The form of f(·) is more specifically related to the spotting apparatus. A simple choice might be un-normalised Gaussian:

$$f(\|m - r_i\|) = \exp\left\{-\frac{1}{2v_i}\|m - r_i\|^2\right\}$$

with $v_i$ appropriately chosen to reflect spot width, and $r_i$ denoting the spot centre. $r_i$ is preferably learned on the basis of the data, without recourse to periodicity considerations, and can depart from the ideal grid. Often, however, a tailored distribution to reflect the unique nature of the spotting device may be more appropriate.

The formulation set out above which defines indicator variable distributions independent of probe quantity can be extended to include probe quantity information.

For on pixels, $\{I_m=1\}$, it can be expected that the probe distribution will evolve in a relatively smooth, or constrained, manner. The form of this distribution is instrumental in the ability of the model to separate valid signal from noise. An example of the information it may be desirable to include would be that given probe concentration is high in all surrounding pixels, it can also be expected that probe concentration will be high in the central pixel on average.

A Markovian field approach is adopted where $d_m$ is considered dependent on the surrounding neighbourhood, and defined through the conditional density $p(d_m|I_{\{m\}}, d_{\{m\}})$ .

In many cases, the neighbourhood can be limited to immediately surrounding values. It can represent, for example, information about edge effects and regions of homogeneity.

In many instances favourable results may still be achieved by assuming $d_m$ drawn independently from a truncated normal, or other simple distribution, parameterised by an unknown scale parameter. This can lead to significant computational advantages.

$$p(d_m|I_m = 1) = N(d_m|0, \lambda)$$

$$p(\lambda) = \lambda^{-1}, (\lambda \geq 0)$$

Information about the consistency of the spotting process, and how much material is being spotted can be used in the invention to improve prior knowledge of this distribution. Parameters of the distribution can be learned by the invention from test data.

Typically, this distribution is again parameterised by a quantity $E[d_m]$ representing the expected spot shape and magnitude. Variance parameters can then be learned to quantify variability in the spotting process, both within and between spots. This is important for absolute quantification of expression levels. It can also be important for quality control tasks.

The following is an example of modelling specific hybridisation.

A certain percentage of the quantity of target $\alpha_m \stackrel{\Delta}{=} \{\alpha_{m,cy3}, \alpha_{m,cy5}\}$ available at each pixel will bind to immobilized probe. The remainder will, under ideal conditions, be washed off.

$a_m$ is therefore related through a complex nonlinear relationship to $a_m$ and $d_m$:

$$\alpha_m = \phi(a_m, d_m, \theta)$$

where $\phi(\cdot)$ is a vector function, $\theta$ potentially includes sequence dependent effects and other unique experimental conditions. This relationship can be empirically derived through experimentation.

Since the amount of DNA bound to the slide is usually far greater than sample concentrations, it is often reasonable to assume $\alpha_m = \phi(d_m, \theta)$. This relationship exhibits some uncertainty. In some instances, direct proportionality with $d_m$ can be appropriate over a certain range.

It is usually reasonable to make the additional assumption that the process relating $\alpha_{m,cy3}$ to $d_m$ and $a_{m,cy3}$ is the same as that relating $\alpha_{m,cy5}$ to $d_m$ and $a_{m,cy5}$ for each spot. As such information is incorporated to exploit the (expected) similarity between spot shapes in cy3 and cy5 channels.

The actual extent of hybridisation is $c_m \sim p(c_m|a_m,\alpha_m)$ where $E[c_m] = a_m \otimes \alpha_m$
This represents additional uncertainty, for example, from the binding process and model assumptions. There are many assumptions that can be made, for example incorporating all variability through $a_m \otimes \alpha_m$. Alternatively, it can be useful to consider $a$, the expected available in each channel across the whole spot, $c_m \sim p(c_m|\alpha,\alpha_m)$, and take variability into account through $p(c_m|\cdot)$.

A well prepared slide will exhibit roughly constant $a_m$ across the entire slide. Exceptions include where wash is uneven (slide level effect), dye separation (local effect). Importantly there is local variability according to target densities at a particular location. For example, if target concentration is on average very low, then some regions will contain no target. A suitable, but not necessary assumption is that over a relatively small region, the mean of the $a_m$ process is fixed. An indicator variable can be used to indicate the presence or absence of target. In this case,

$$a_m \sim p(a_m|E[a_m],V[a_m])$$

where for example $p(\cdot)$ is an Inverted Gamma or Gamma distribution ensuring positivity. $E[a_m]$ is constant and indicative of the expected concentration of target at each pixel in each channel (or the total overall in the region). It does not specifically try to model clumping effects, but certainly can include them. $E[a_m]$ and $V[a_m]$ can both be learned from the data with appropriate constraints on form of distribution and parameter ranges. This distribution can be made more complicated to represent information about how true underlying quantity $E[a_m]$ gets transformed into $\{a_m\}$ through a variability parameter. By estimating $V[a_m]$ it is possible to understand variability in $E[a_m]$, one of the key inference qualities in an analysis. This applies for donut shapes and so forth, where the shape may imply a high variability parameter.

Alternatively wavelets, splines, or other functions capable of modelling slowly varying effects can be also used.

Non-specific hybridisation across the slide can be caused by factors such as incomplete blocking and dye removal.

Variation in the non-specific hybridization process (to the slide as opposed to the probe) is typically slow; block stationarity can be a reasonable assumption. Existing literature regularly assumes piecewise constant or linear background.

The process is actually more complicated. We consider a model of the form:

$$b_{m,cy3} \; p(b_{m,cy3}|b_{-m,}I_{m,}d_m,a_m)$$

Note that $p(b_{m,cy3}|b_{-m,}d_m,a_m)$ is dependent on the presence of probe DNA which can reduce non-specific hybridization (as potentially can target DNA). A suitable distribution to represent the background, with its probe dependence, is a standard Gaussian MRF where the mean at a particular location is dependent on both the surrounding background values and the parameters $\{I_m, d_m, a_m\}$. An example would be an expected halving in background hybridization in areas with high probe concentrations, relative to what would otherwise be predicted by the MRF.

Non-specific hybridization can also occur when imperfect hybridisation leads to two similar but not identical target sequences binding to the same probe sequence. If two probe sequences are similar, or something of the target composition is known, this non-specific hybridisation can be predicted. Moreover, dependent on the difference between the sequences, it can be relatively precisely characterised. For example a model where the difference between sequences is exponentially related to the non-specific hybridisation potential can be useful.

The models described above are suitable for a single spot. However, since the total number of spots is known thereby avoiding certain model selection difficulties, it is straightforward to expand the system to include the possibility of multiple overlapping spots.

The number of photons emitted is dependent on a number of factors including, most importantly, the extent of hybridisation, the strength of the laser and the sequence dependent fluorescent emission characteristics of the dyes in question. It is an uncertain quantity. In reality, this is expected to be approximately Poisson distributed. Alternative formulations can be devised. These photon numbers are then measured through a potentially nonlinear photon multiplier device, which introduces its own noise (this additive noise also encapsulates thermal noise etc. which can be considered independent of signal). Contributions may be encountered from adjacent pixels (convolution). The total measurement is thus

$$\boldsymbol{y}_m = \boldsymbol{v}(h * \boldsymbol{f}_{(m)} + \boldsymbol{n}_m)$$

where $\boldsymbol{f}_{\{m\}}$ denotes the photon emission, * denotes the convolution operator, $h$ denotes a fixed mixing function dependent on the scanner and apparatus in question, and $v(\cdot)$ represents the nonlinear photon multiplier device. Importantly $v(\cdot)$ can also be used to model offset between the channels owing to scanner alignment issues. This can alternatively be represented as a matrix multiplication. Information on $h(\cdot)$ is usually well understood by scanner manufacturers, but can be learned from the data if required.

Then : $\boldsymbol{f}_m \sim P(\boldsymbol{c}_m\omega)$

where $\omega$ is a sequence dependent gain constant also dependent on the unique resonance formed through binding of the fluorescent dye to the target, the laser strength, and potentially other factors. $\omega$ can be treated as uncertain, and prior knowledge about the effect of sequence, fluorescent dye, and laser strength included.

Alternative approximating formulations may be employed by the invention. Some with computational advantage, could include

$$\boldsymbol{f}_m = \boldsymbol{c}_m\omega \quad \text{or} \quad \boldsymbol{f}_m = \sqrt{\boldsymbol{c}_m}\,\omega$$

where uncertainty in $\omega$ models photon emission noise and other signal dependent parts of the emission process. The dependence of photon emission noise on signal strength is maintained. Typical distributions for $\omega$ include Gamma, Inverted Gamma, and Gaussian distributions.

The remaining noise $\boldsymbol{n}_m$ is assumed independent between the cy3 and cy5 channels. It may be Gaussian, or from a distribution ensuring positivity such as the Gamma or Inverted Gamma distributions. The variance and mean of the process are typically considered static but unknown. Other parameterisations are similar.

The models are sufficiently powerful to make meaningful predictions of missing data. Missing data can occur with saturation of the scanning device (leading to readouts at the top of the scanner range), or scratches (leading to zero readouts). Missing data is relatively trivial to detect. Of particular relevance to the estimation are values in the non-saturated channel and the expected shape distribution. (Saturation regularly occurs in one channel only. However, in fact because of the interaction between non-specific hybridisation and bound DNA, even if there is no target this can be deduced.)

Saturation is represented through $v(\cdot)$. Simply $v(\cdot)$ is equal to the top of the scanner range for values above the saturation threshold. Standard Markov chain Monte Carlo methods, among others, can be used in combination with the models just described to perform inference.

**Claims**

1. A method of analysing microarray images, the method comprising the steps of:

   receiving data from a microarray process,
   modelling the microarray process to define a microarray model comprising at least one of a target distribution of test and control sample sequences and defining a first independent sub-model and a probe distribution of a known sample sequence and defining a second independent sub-model,
   comparing the received data with the microarray model in order to extract information from the data, and
   outputting the information.

2. A method according to claim 1, wherein the data is received from a detector corresponding to a control target sample and a detector corresponding to a test target sample.

3. A method according to claim 2, wherein the model includes information about statistical similarity in the spot profile corresponding to each detector due to the spot profiles being formed from a common probe.

4. A method according to any preceding claim, wherein the microarray process is a DNA microarray process.

5. A method according to any preceding claim, wherein the extracted information is gene expression information.

6. A method according to any preceding claim, wherein when at least the second independent sub-model is employed in the modelling step, the second independent sub-model comprises a model of the spotting process.

7. A method according to claim 6, wherein the model of the spotting process includes an understanding of how adjacent spots interact.

8. A method according to any preceding claim, wherein the modelling step further comprises modelling the interaction between the background distribution of the received signal and at least one of target distribution and probe distribution.

9. A method according to claim 8, wherein the background distribution includes non-specific hybridisation.

10. A method according to any preceding claim, wherein the modelling step further comprises modelling fluorescence to define a third independent sub-model.

11. A method according to claim 10, wherein the third independent sub-model includes information on the effect of DNA sequence on fluorescence.

12. A method according to any preceding claim, wherein the modelling step further comprises modelling hybridisation to define a fourth independent sub-model.

13. A method according to claim 12, wherein the fourth independent sub-model includes information on the effect of sequence on hybridisation.

14. A method according to any preceding claim, wherein the modelling step further comprises modelling spatial variation of target concentration.

15. A method according to any preceding claim, wherein the comparing step further comprises comparing the received image data with the microarray model in order to predict missing data.

16. A method according to claim 15, wherein the missing data is due to saturation in the device which creates the image data.

17. A method according to any preceding claim, wherein the modelling step further comprises modelling detector nonlinearity.

18. A method according to any preceding claim, wherein the structure of the microarray model is hierarchical.

19. A method according to any preceding claim, wherein the data received from the microarray process is image data.

20. A method according to any of claims 1 to 18, wherein the data received from the microarray process is pre-analysed data.

21. A method according to any preceding claim, wherein standard Markov chain Monte Carlo methods are employed.

22. An apparatus for analysing microarray images, the apparatus comprising:

> means for receiving data from a microarray process,
> means for modelling the microarray process to define a microarray model comprising at least one of a target distribution of test and control sample sequences defining a first independent sub-model and probe distribution of a known sample sequence and defining a second independent sub-model,
> means for comparing the received data with the microarray model in order to extract information from the data, and
> means for outputting the information.

23. An apparatus according to claim 22, wherein the data is received from a channel corresponding to a control target

**EP 1 408 323 B1**

sample and a channel corresponding to a test target sample.

24. An apparatus according to claim 22 or claim 23, wherein the microarray process is a DNA microarray process.

25. An apparatus according to any of claims 22 to 24, wherein the extracted information is gene expression information.

26. An apparatus according to any of claims 22 to 25, wherein the means for modelling further comprises means for modelling the interaction between the background distribution of the received signal and at least one of target distribution and probe distribution.

27. An apparatus according to any claims 22 to 26, wherein the means for modelling further comprises means for modelling fluorescence to define a third independent sub-model.

28. An apparatus according to any of claims 22 to 27, wherein the means for modelling further comprises means for modelling hybridisation to define a fourth independent sub-model.

29. An apparatus according to any of claims 22 to 28, wherein the means for modelling further comprises means for modelling spatial variation of target concentration.

30. An apparatus according to any claims 22 to 29, wherein the means for comparing further comprises means for comparing the received image data with the microarray model in order to predict missing data.

31. An apparatus according to any of claims 22 to 30, wherein the means for modelling further comprises means for modelling detector nonlinearity.

32. An apparatus according to any of claims 22 to 31 wherein the data received from the microarray process is image data.

33. An apparatus according to any of claims 22 to 31, wherein the data received from the microarray process is pre-analysed data.

**Patentansprüche**

1. Verfahren zum Analysieren von Mikroarray-Bildern, wobei das Verfahren die Schritte umfasst:

   Empfangen von Daten von einem Mikroarray-Prozess,
   Modellieren des Mikroarray-Prozesses, um ein Mikroarray-Modell zu definieren, das zumindest eine Verteilung einer Zielverteilung von Test- und Kontrollprobensequenzen, die ein erstes unabhängiges Untermodell definiert, und einer Untersuchungsverteilung einer bekannten Probensequenz, die ein zweites unabhängiges Untermodell definiert, umfasst,
   Vergleichen der empfangenen Daten mit dem Mikroarray-Modell, um Informationen aus den Daten zu gewinnen, und Ausgeben der Informationen.

2. Verfahren nach Anspruch 1, wobei die Daten von einem Detektor, der zu einer Kontrollzielprobe korrespondiert, und einem Detektor, der zu einer Testzielprobe korrespondiert, empfangen werden.

3. Verfahren nach Anspruch 2, wobei das Modell Informationen über eine statistische Ähnlichkeit in dem Erkennungsprofil, das jedem Detektor entspricht, umfasst, da die Erkennungsprofile aus einer gemeinsamen Untersuchung gebildet werden.

4. Verfahren nach einem vorhergehenden Anspruch, wobei der Mikroarray-Prozess ein DNA-Mikroarray-Prozess ist.

5. Verfahren nach einem vorhergehenden Anspruch, wobei die gewonnenen Informationen Genexpressionsinformationen sind.

6. Verfahren nach einem der vorhergehenden Ansprüche, wobei, wenn zumindest das zweite unabhängige Untermodell im Modellierschritt verwendet wird, das zweite unabhängige Untermodell ein Modell des Erkennungsprozesses umfasst.

9

7. Verfahren nach Anspruch 6, wobei das Modell des Erkennungsprozesses ein Verständnis umfasst, wie benachbarte Punkte interagieren.

8. Verfahren nach einem vorhergehenden Anspruch, wobei der Modellierschritt ferner ein Modellieren der Interaktion zwischen der Hintergrundverteilung des empfangenen Signals und der Zielverteilung und/oder der Untersuchungsverteilung umfasst.

9. Verfahren nach Anspruch 8, wobei die Hintergrundverteilung eine nicht-spezifische Hybridisierung umfasst.

10. Verfahren nach einem vorhergehenden Anspruch, wobei der Modellierschritt ferner ein Modellieren von Fluoreszenz umfasst, um ein drittes unabhängiges Untermodell zu definieren.

11. Verfahren nach Anspruch 10, wobei das dritte unabhängige Untermodell Informationen über die Wirkung der DNA-Sequenz auf die Fluoreszenz umfasst.

12. Verfahren nach einem vorhergehenden Anspruch, wobei der Modellierschritt ferner ein Modellieren der Hybridisierung umfasst, um ein viertes unabhängiges Untermodell zu definieren.

13. Verfahren nach Anspruch 12, wobei das vierte unabhängige Untermodell Informationen über die Wirkung der Sequenz auf die Hybridisierung umfasst.

14. Verfahren nach einem vorhergehenden Anspruch, wobei der Modellierschritt ferner ein Modellieren einer räumlichen Veränderung der Zielkonzentration umfasst.

15. Verfahren nach einem vorhergehenden Anspruch, wobei der Vergleichsschritt ferner ein Vergleichen der empfangenen Bilddaten mit dem Mikroarry-Modell umfasst, um fehlende Daten vorauszuberechnen.

16. Verfahren nach Anspruch 15, wobei die Daten aufgrund einer Sättigung in der Vorrichtung, welche die Bilddaten erzeugt, fehlen.

17. Verfahren nach einem vorhergehenden Anspruch, wobei der Modellierschritt ferner ein Modellieren der Detektor-Nichtlinearität umfasst.

18. Verfahren nach einem vorhergehenden Anspruch, wobei die Struktur des Mikroarray-Modells hierarchisch ist.

19. Verfahren nach jedem vorhergehenden Anspruch, wobei die vom Mikroarray-Prozess empfangenen Daten Bilddaten sind.

20. Verfahren nach einem der Ansprüche 1 to 18, wobei die vom Mikroarray-Prozess empfangenen Daten voranalysierte Daten sind.

21. Verfahren nach einem vorhergehenden Anspruch, wobei übliche Markow-Chain-Monte-Carlo-Verfahren verwendet werden.

22. Vorrichtung zum Analysieren von Mikroarray-Bildern, wobei die Vorrichtung umfasst:

    eine Einrichtung zum Empfangen von Daten von einem Mikroarray-Prozess,
    eine Einrichtung zum Modellieren des Mikroarray-Prozesses, um ein Mikroarray-Modell zu definieren, das zumindest eine Verteilung einer Zielverteilung von Test- und Kontrollprobensequenzen, die ein erstes unabhängiges Untermodells definiert, und einer Untersuchungsverteilung einer bekannten Probensequenz, die ein zweites unabhängiges Untermodell definiert, umfasst,
    eine Einrichtung zum Vergleichen der empfangenen Daten mit dem Mikroarray-Modell, um Informationen aus den Daten zu gewinnen, und
    eine Einrichtung zum Ausgeben der Informationen.

23. Vorrichtung nach Anspruch 22, wobei die Daten von einem Kanal, der zu einer Kontrollzielprobe korrespondiert, und einem Kanal, der zu einer Testzielprobe korrespondiert, empfangen werden.

24. Vorrichtung nach Anspruch 22 oder Anspruch 23, wobei der Mikroarray-Prozess ein DNA-Mikroarray-Prozess ist.

25. Vorrichtung nach einem der Ansprüche 22 bis 24, wobei die gewonnenen Informationen Genexpressionsinformationen sind.

26. Vorrichtung nach einem der Ansprüche 22 bis 25, wobei die Einrichtung zum Modellieren ferner eine Einrichtung zum Modellieren der Interaktion zwischen der Hintergrundverteilung des empfangenen Signals und der Zielverteilung und/oder der Untersuchungsverteilung umfasst.

27. Vorrichtung nach einem der Ansprüche 22 bis 26, wobei die Einrichtung zum Modellieren ferner eine Einrichtung zum Modellieren von Fluoreszenz umfasst, um ein drittes unabhängiges Untermodell zu definieren.

28. Vorrichtung nach einem der Ansprüche 22 bis 27, wobei die Einrichtung zum Modellieren ferner eine Einrichtung zum Modellieren der Hybridisierung umfasst, um ein viertes unabhängiges Untermodell zu definieren.

29. Vorrichtung nach einem der Ansprüche 22 bis 28, wobei die Einrichtung zum Modellieren ferner eine Einrichtung zum Modellieren einer räumlichen Veränderung der Zielkonzentration umfasst.

30. Vorrichtung nach einem der Ansprüche 22 bis 29, wobei die Einrichtung zum Vergleichen ferner eine Einrichtung zum Vergleichen der empfangenen Bilddaten mit dem Mikroarray-Modell umfasst, um fehlende Daten vorauszuberechnen.

31. Vorrichtung nach einem der Ansprüche 22 bis 30, wobei die Einrichtung zum Modellieren ferner eine Einrichtung zum Modellieren der Detektor-Nichtlinearität umfasst.

32. Vorrichtung nach einem der Ansprüche 22 bis 31, wobei die vom Mikroarray-Prozess erhaltenen Daten Bilddaten sind.

33. Vorrichtung nach einem der Ansprüche 22 bis 31, wobei die vom Mikroarray-Prozess erhaltenen Daten voranalysierte Daten sind.

**Revendications**

1. Procédé d'analyse d'images de microéchantillon, le procédé comprenant les étapes consistant à :

   recevoir des données provenant d'un processus de microéchantillon,
   modéliser le processus de microéchantillon pour définir un modèle de microéchantillon comprenant au moins une distribution de cible des séquences d'échantillons d'essai et de contrôle et définissant un premier sous-modèle indépendant et une distribution de sonde d'une séquence d'échantillons connue et définissant un deuxième sous-modèle indépendant,
   comparer les données reçues au modèle de microéchantillon afin d'extraire des informations à partir des données, et
   produire les informations.

2. Procédé selon la revendication 1, dans lequel les données sont reçues d'un détecteur correspondant à un échantillon cible de contrôle et un détecteur correspondant à un échantillon cible d'essai.

3. Procédé selon la revendication 2, dans lequel le modèle comprend des informations sur la similarité statistique dans le profil de dépôt correspondant à chaque détecteur due au fait que les profils de dépôt sont formés à partir d'une sonde commune.

4. Procédé selon l'une quelconque des revendications précédentes, dans lequel le processus de microéchantillon est un processus de microéchantillon ADN.

5. Procédé selon l'une quelconque des revendications précédentes, dans lequel les informations extraites sont des informations d'expression de gène.

**6.** Procédé selon l'une quelconque des revendications précédentes, dans lequel, lorsqu'au moins le second sous-modèle indépendant est employé dans l'étape de modélisation, le second sous-modèle indépendant comprend un modèle du processus de dépôt.

**7.** Procédé selon la revendication 6, dans lequel le modèle du processus de dépôt comprend une compréhension de la manière dont interagissent les dépôts adjacents.

**8.** Procédé selon l'une quelconque des revendications précédentes, dans lequel ladite étape de modélisation comprend en outre la modélisation de l'interaction entre la distribution d'arrière-plan du signal reçu et au moins une de la distribution de cible et de la distribution de sonde.

**9.** Procédé selon la revendication 8, dans lequel la distribution d'arnère-plan comprend une hybridation non spécifique.

**10.** Procédé selon l'une quelconque des revendications précédentes, dans lequel l'étape de modélisation comprend en outre la modélisation de la fluorescence pour définir un troisième sous-modèle indépendant.

**11.** Procédé selon la revendication 10, dans lequel le troisième sous-modèle indépendant comprend des informations sur l'effet de la séquence ADN sur la fluorescence.

**12.** Procédé selon l'une quelconque des revendications précédentes, dans lequel l'étape de modélisation comprend en outre la modélisation de l'hybridation pour définir un quatrième sous-modèle indépendant.

**13.** Procédé selon la revendication 12, dans lequel le quatrième sous-modèle indépendant comprend des informations sur l'effet de la séquence sur l'hybridation.

**14.** Procédé selon l'une quelconque des revendications précédentes, dans lequel l'étape de modélisation comprend en outre la modélisation de la variation spatiale de la concentration cible.

**15.** Procédé selon l'une quelconque des revendications précédentes, dans lequel l'étape de comparaison comprend en outre la comparaison des données d'image reçues avec le modèle de microéchantillon afin de prédire les données manquantes.

**16.** Procédé selon la revendication 15, dans lequel les données manquantes sont dues à la saturation dans le dispositif qui crée les données d'image.

**17.** Procédé selon l'une quelconque des revendications précédentes, dans lequel l'étape de modélisation comprend en outre la modélisation de la non-linéarité du détecteur.

**18.** Procédé selon l'une quelconque des revendications précédentes, dans lequel la structure du modèle de microéchantillon est hiérarchique.

**19.** Procédé selon l'une quelconque des revendications précédentes, dans lequel les données reçues du processus de microéchantillon sont des données d'image.

**20.** Procédé selon l'une quelconque des revendications 1 à 18, dans lequel les données reçues à partir du processus de microéchantillon sont des données préanalysées.

**21.** Procédé selon l'une quelconque des revendications précédentes, dans lequel les méthodes Monte Carlo par chaînes de Markov standards sont employées.

**22.** Appareil pour analyser des images de microéchantillon, l'appareil comprenant :

des moyens pour recevoir des données provenant d'un processus de microéchantillon,
des moyens pour modéliser le processus de microéchantillon pour définir un modèle de microéchantillon comprenant au moins une d'une distribution de cible des séquences d'échantillons d'essai et de contrôle et définissant un premier sous-modèle indépendant et une distribution de sonde d'une séquence d'échantillons connue et définissant un deuxième sous-modèle indépendant,
des moyens pour comparer les données reçues avec le modèle de microéchantillon afin d'extraire des informations des données, et
des moyens pour produire les informations.

23. Appareil selon la revendication 22, dans lequel les données sont reçues à partir d'un canal correspondant à un échantillon cible de contrôle et un canal correspondant à un échantillon cible d'essai.

24. Appareil selon la revendication 22 ou la revendication 23, dans lequel le processus de microéchantillon est un processus de microéchantillon ADN.

25. Appareil selon l'une quelconque des revendications 22 à 24, dans lequel les informations extraites sont des informations d'expression de gènes.

26. Appareil selon l'une quelconque des revendications 22 à 25, dans lequel les moyens de modélisation comprennent en outre des moyens pour modéliser l'interaction entre la distribution d'arrière-plan du signal reçu et au moins une de la distribution de cible et de la distribution de sonde.

27. Appareil selon l'une quelconque des revendications 22 à 26, dans lequel les moyens de modélisation comprennent en outre des moyens pour modéliser la fluorescence pour définir un troisième sous-modèle indépendant.

28. Appareil selon l'une quelconque des revendications 22 à 27, dans lequel les moyens de modélisation comprennent en outre des moyens pour modéliser l'hybridation pour définir un quatrième sous-modèle indépendant.

29. Appareil selon l'une quelconque des revendications 22 à 28, dans lequel les moyens de modélisation comprennent en outre des moyens pour modéliser la variation spatiale de la concentration cible.

30. Appareil selon l'une quelconque des revendications 22 à 29, dans lequel les moyens de comparaison comprennent en outre des moyens pour comparer les données d'image reçues avec le modèle de microéchantillon afin de prédire les données manquantes.

31. Appareil selon l'une quelconque des revendications 22 à 30, dans lequel les moyens de modélisation comprennent en outre des moyens pour modéliser la non-linéarité du détecteur.

32. Appareil selon l'une quelconque des revendications 22 à 31, dans lequel les données reçues du processus de microéchantillon sont des données d'image.

33. Appareil selon l'une quelconque des revendications 22 à 31, dans lequel les données reçues du processus de microéchantillon sont des données préanalysées.

Figure 1

9

20

21

22

Fig. 2